# EUROPEAN PATENT APPLICATION

(11) **EP 1 508 623 A1**
(43) Date of publication of application: **23.02.2005**
(21) Application number: 03700566.7
(22) Date of filing: 15.01.2003
(51) Int. Cl.: C12Q 1/68, C12N 15/09, G01N 33/50

(54) **METHOD OF SPECIFYING SNP**

(30) Priority: 15.01.2002 JP 2002042355; 23.07.2002 JP 2002213695
(71) Applicant: GENESYS TECHNOLOGIES, INC., Chuo-ku, Tokyo 103-0023 (JP)
(72) Inventor: SUZUKI, Toshiaki, Fujisawa-shi, Kanagawa 251-0015 (JP); KAMATANI, Naoyuki, Kunitachi-shi, Tokyo 186-0005 (JP); TANAKA, Junji, Kamakura-shi, Kanagawa 247-0071 (JP)
(74) Representative: Möbus, Steffen
(86) International application number: PCT/JP2003/000261
(87) International publication number: WO 2003/060162

(57) **Abstract**

(1) It is intended to provide a technique relating to a method of specifying an SNP which comprises repeating presumption of an SNP serving as a marker and detailed typing of SNPs around the same, thus gradually narrowing down the focus to the base sequence domain in which the 'target' SNP is likely contained and finally specifying the 'target' SNP at a high efficiency. As Fig. 1 shows, the method of specifying an SNP comprises: (1) determining a drug to be developed which is the subject of the determination; (2) collecting samples to be analyzed; (3) determining a 'scanning domain (base sequence domain)'; (4) determining 'typing' SNPs; (5) SNP typing by the wet process and analyzing haplotypes based on the typing data; (6) presuming a 'marker' SNP (determining the analytical data); and (7) specifying the 'target' SNP (target SNP). A cycle consisting of the stage (1) to (7) is repeated as a treatment cycle.

## Description

### BACKGROUND OF THE INVENTION

### Field of the Invention:

This invention relates to a method of specifying SNP for drug responsiveness that employs SNP function analysis techniques for the improvement of the efficiency and accuracy of the SNP function analysis process, and for use in clinical trials of newly developed drugs by the drug industry.

### Description of the Related Art:

In the conventional SNP function analysis process that specifies the SNP (Single Nucleotide Polymorphism--single nucleotide polymorphism or position of a single nucleotide polymorphism) related to disease susceptibility or drug responsiveness, SNP typing by a wet process (Note 1) was performed after narrowing down the SNP to be analyzed to several tens or several thousands of locations due to cost.

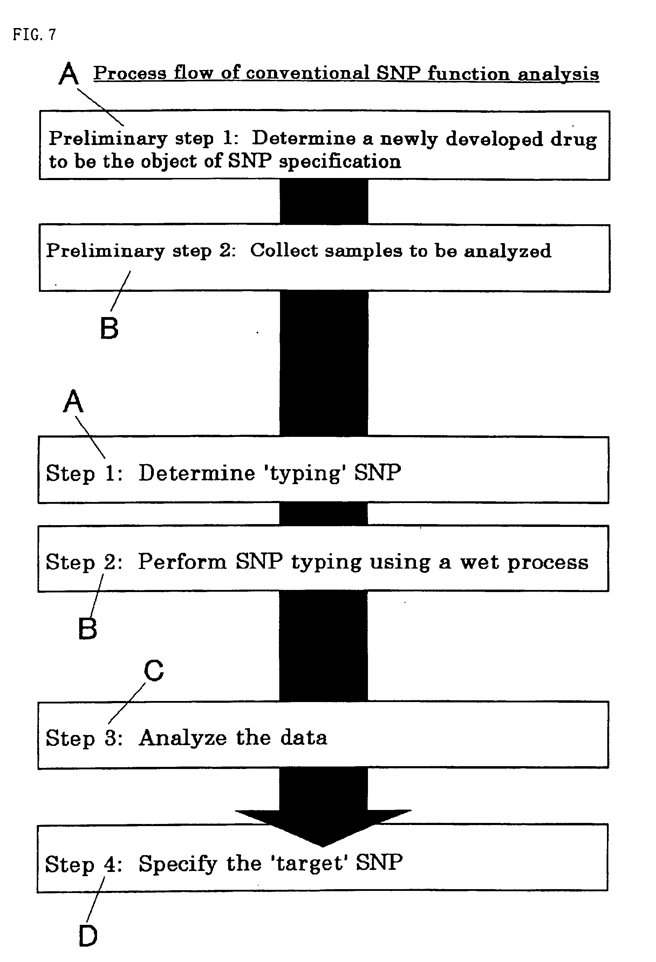

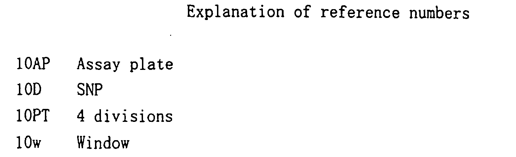

Fig. 7 is a drawing showing the flow of the conventional SNP function analysis process. As shown in Fig. 7, conventional SNP function analysis was performed in the order of preliminary step A (determining of new drug to be developed), preliminary step B (collecting samples to be analyzed), step A (setting typing SNP), step B (SNP typing by a wet process), step C (data analysis) and step D (specifying the 'target' SNP).

This is because the cost (chemical costs per sample) in the case of typing all of the 3 million human SNP using the TaqMan method (Note 2) is approximately 200 million yen, and in order to perform SNP typing for the several hundred or several thousand of samples necessary for statistical analysis of the SNP function, unrealistic costs such as several 10 to several 100 billion yen, and resources such as large-scale analysis facilities are necessary.

Therefore, in the normal SNP function analysis process, the SNP to be typed (hereafter referred to as typing SNP) are limited, and function analysis is performed after narrowing down the SNP to a 1000 to 10, 000 SNP.

### (Pg. 2)

However, there is no other method to determine whether or not there is a relationship between disease susceptibility or drug responsiveness and SNP than by statistical determination from the results of typing those SNP. Therefore, the 'target' SNPs (Note 3), which are finally determined to be related, must be included in and selected from a group of 1,000 to 10,000 SNP beforehand as typing SNP. In the case that these SNP are not selected, the related SNP cannot be found in the analysis, and so the analysis process must be performed again from selection of a group of typing SNP.

In the conventional method of selecting typing SNP, the researcher used a technique of searching reference documents such as research papers and genome-related databases, and performing a homological search that predicts the function of human genes that are similar to genomes that are not human whose functions area already known.

However, the functions of human genomes are not completely given in this genome information. Therefore, the step of selecting typing SNP that determine the efficiency of this SNP function analysis process, or in other words, whether or not it is possible to predict a 'target' SNP at a high probability, depends largely on the experience and skill of the researcher as well as luck.

Also, one more problem with the SNP function analysis process is the quality of data. In the SNP function analysis, SNP typing is performed between sample groups that are classified according to whether or not they express certain characteristics (for example, behavior or susceptibility), and the frequency of allele of each of the SNP of both groups is analyzed statistically, and the SNP that causes that characteristic to be expressed is identified. In other words, when there is a problem with the quality of typing data of the wet process, the results of SNP function analysis based on that data become inaccurate.

This problem is due to the fact that SNP typing is a process with human intervention. Many of the quality problems that are inherent in the SNP function analysis process, such as contamination and careless mistakes in operation such as mixing up samples and reagents that cause a drop in quality of the data are human related, and quality is largely dependent on these and the experience and skill of the researcher.

### (Pg. 3)

(Note 1) The wet process is a process for performing SNP typing. In the current TaqMan method, gene samples of blood or the like are caused to react with a reagent on a plate and hybridization is performed, and the results are optically measured, and then finally, typing of the allele of the samples is performed using that SNP. This process is called a wet process. Statistical analysis of the specified typing data is not included in the wet process.

(Note 2) This is a typing method that uses the PCR (Polymerase Chain Rereaction) reaction between fluorescent-labeled allele specific oligo and Taq DNA polymerase.

(Note 3) The 'target' SNPs or SNPs that will become the 'target' are either SNPs that cause the disease susceptibility or drug responsiveness (of newly developed drugs), or SNPs that are indicators or markers of disease susceptibility or drug responsiveness. The object of the SNP function analysis is to specify these SNPs.

However, there were the following problems in the conventional technology.

There are problems in that predicting 'target' SNPs before typing and properly and accurately selecting a group of several hundred or several thousand SNP that include these is difficult, and preventing occurrences of mixed up samples or reagents and contamination due to human error during the wet process that lower the quality of the SNP typing data is extremely difficult.

### SUMMARY OF THE INVENTION

Taking the aforementioned problems into consideration, the object of the present invention is to provide a method of specifying SNP that 1) gradually narrows down the base sequence domain in which it is thought that the 'target' SNP exists, and finally specifies the 'target' SNP efficiently by repeatedly estimating a SNP as a marker and performing detailed typing of the SNPs near that marker, and 2) compares statistics for patients and non-patients and narrows down the SNP domain by a process-control method that prevents careless mistakes in operation that cause a drop in data quality in the wet process and by eliminating any data that were contaminated by contamination or the like before performing statistical analysis.

The invention according to a first claim is a method of specifying SNP related to disease susceptibility or drug responsiveness and comprising: a first step of setting a scanning domain beforehand in the base sequence domain that is the object of SNP analysis; a second step of gradually narrowing down the scanning domain to a localized domain that contains a target SNP; and a third step of specifying the target SNP from the narrowed down localized domain.

The invention according to a second claim is the method of specifying SNP of claim 1 in which the second step comprises a step of setting a marker SNP for specifying the target SNP and gradually narrowing down the scanning domain.

The invention according to a third claim is the method of specifying SNP of the second claim in which the second step uses statistical analysis such as haplotype analysis to set the marker SNP.

The invention according to a fourth claim is the method of specifying SNP of claim 3 in which the first step comprises: a step of setting the scanning domain of the base sequence domain in a genome domain that is limited to genes whose functions are clearly known or chromosomes whose functions can be predicted; and the second step comprises: a fourth step of selecting a group of SNP to be typed from the scanning domain and performing SNP typing using a wet process; a fifth step of finding the probability of appearance of all combinations of the haplotype analysis in the scanning domain based on typing data of the SNP typing as a statistical amount; and a sixth step of comparing the found statistical amount with a preset or estimated reference statistical amount, and when there is significant deviation between the statistical amount and the reference statistical amount that exceeds a preset threshold, determining that the marker SNP is contained in the domain corresponding to the deviated position that exceeds the threshold value.

### (Pg. 5)

The invention according to a fifth claim is the method of specifying SNP of claim 4 in which the third step comprises: a seventh step of increasing the specified ratio of the number of SNPs to be the object of typing in the selection of the SNP group in the fourth step when the significant deviation is less than a first threshold value, and then repeating the fifth step; an eighth step of setting a new scanning domain from the scanning domain that has been decreased by a specified ratio such that it contains the position of the deviated peak when the significant deviation is greater than the first threshold value but less than a second threshold value, and then repeating the fifth step; and a ninth step of determining that the marker SNP is contained in the domain corresponding to the deviated position that exceeds the second threshold value when the significant deviation exceeds the second threshold value, setting a new scanning domain from the scanning domain that has been decreased by a specified ratio such that it contains the position of the deviated peak, and then repeating the fifth step.

The invention according to a sixth claim is the method of specifying SNP of claim 5 in which the ninth step comprises a step of setting SNPs that include the target SNP for which all DNA samples are typed when the number of SNPs in a selected group is less then a specified number.

The invention according to a seventh claim is the method of specifying SNP of claim 5 in which the seventh step comprises a step of determining that the target SNP is not contained and stopping the process when the number of times the process of the fifth step is performed exceeds a specified number of times.

The invention according to an eighth claim is the method of specifying SNP of claim 5 in which the eighth step comprises a step of determining that the target SNP is not contained and stopping the process when the number of times the process of the fifth step is performed exceeds a specified number of times.

### (Pg. 6)

The invention according to a ninth claim is the method of specifying SNP of any one of the claims 1 thru 8 in which the second step comprises a step of typing the SNP using a quality controlled process, and where the quality controlled process performs typing of four SNP on one assay plate for one sample, and determines that the typing data is invalid when the number of typed SNPs found having significant difference by a statistical method such as the Chi-square test exceeds a specified number and identifies the data as being contaminated by contamination of the sample.

The invention according to a tenth claim is the method of specifying SNP of claim 9 in which the second step repeats SNP typing only for SNP found to have significant difference when the number of typed SNPs found to have significant difference was a specified number, and when the result of no significant difference continues for a specified number of times, determines that the typing data is correct and uses that data.

The invention according to an eleventh claim is a computer program that can be read by a computer that can execute the processing of the method of specifying SNP of any one of the claims 1 thru 10 in which all of the steps of any one of the claims 1 thru 10 are coded.

### BRIEF EXPLANATION OF THE DRAWINGS

Fig. 1 is a process flowchart showing the method of specifying SNP of a first embodiment of the invention.
Figs. 2A to 2C are drawings showing steps 3, 4 and 6 of Fig. 1 in detail.
Fig. 3 is a drawing showing an example of the sample and reagent tubes, various plates and related data schemes for step S5 in Fig. 1.
Fig. 4 is a drawing showing an example of the arrangement pattern of SNP on the assay plate 10AP in Fig. 3.
Fig. 5 is a drawing showing an example of the analysis data of step S7 (step 5) in Fig. 1.
Fig. 6 is a drawing showing an example of the analysis data of step S7 in Fig. 1.
Fig. 7 is a drawing showing the process flow of conventional SNP function analysis.

### (Pg. 7)

### DESCRIPTION OF THE PREFERRED EMBODIMENTS

The preferred embodiments of the present invention are explained below based on the drawings.

### (Embodiment 1)

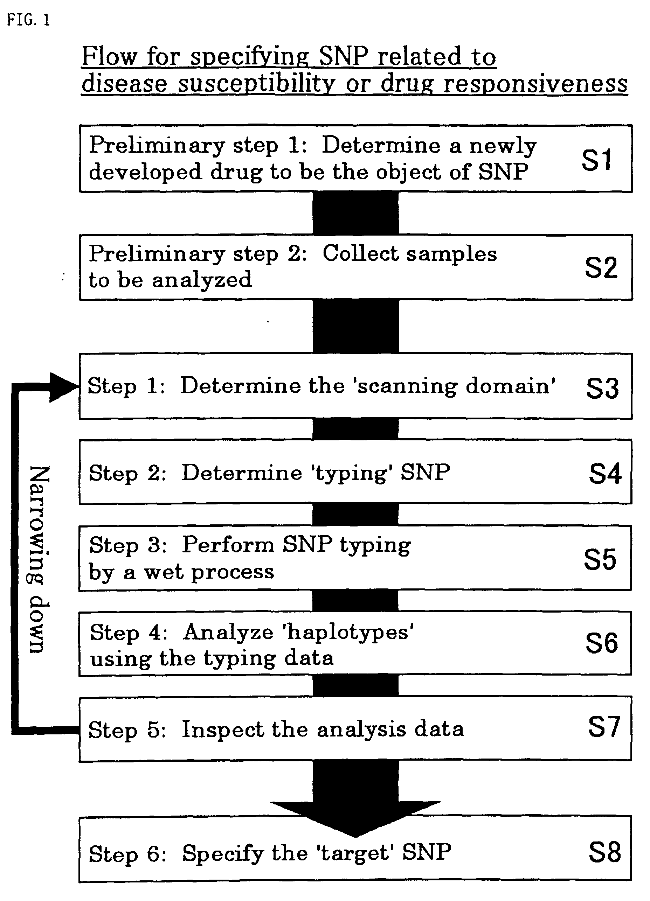

Fig. 1 is a process flowchart showing the method of specifying SNP of a first embodiment of the invention. As shown in Fig. 1, the method of specifying SNP of this first embodiment comprises: a step (step S1) of determining a drug to be newly developed for which the SNP will be specified, a step (step S2) of collecting samples to be analyzed, a step (step S3) of determining the 'scanning domain (base sequence domain)', a step (step S4) of determining 'typing' SNP, a step (step S5) of performing SNP typing by a wet process, a step (step S6) of analyzing haplotypes based on the typing data, a step (step S7) of estimating a 'marker' SNP (determining analysis data) and a step (step S8) of specifying a 'target' SNP (target SNP), and wherein steps S3 to S7 are repeated as one cycle (processing cycle).

With specifying SNP related to disease susceptibility or drug responsiveness as the object, in this process model, by performing the following eight processes (steps), the 'scanning domain' for which SNP typing is performed is gradually narrowed down, and finally the 'target' SNP related to whether or not there is drug responsiveness to the newly developed drug is specified.

The eight steps are described below. First, preliminary step 1 is a step (step S1) of determining a drug to be newly developed for which the SNP will be specified. Preliminary step 2 is a step (step S2) of collecting samples to be analyzed. Step 1 is a step (step S3) of determining the 'scanning domain' (sets the scanning domain beforehand). Step 2 is a step (step S4) of determining 'typing' SNP (typing SNP). Step 3 is a step (step S5) of performing SNP typing by a wet process. Step 4 is a step (step S6) of analyzing haplotypes based on the typing data. Step 5 is a step (step S7) of estimating a 'marker' SNP (determining analysis data). Step 6 is a step (step S8) of specifying a 'target' SNP. Of these eight steps, step 1 to step 5 (steps S3 to S7) are repeated as one cycle (processing cycle).

### (Pg. 8)

Next, the processing by each of the steps will be explained in detail with reference to Fig. 1.

(a) Preliminary step 1 (determining a newly developed drug for SNP specification): A drug is selected from among new drugs developed by the drug industry for which the drug responsiveness (whether or not the drug is effective or has side effects) of the newly developed drug has been tested using SNP. Also, in this process model, it is possible to check the relationship with SNP using disease susceptibility as the object for example.

(b) Preliminary step 2 (collect a sample to analyze): In the SNP function analysis, SNP data are compared between sample groups that are separated according to whether or not a certain characteristic is expressed, and the SNP that causes that characteristic to be expressed is specified (step S2). For example, in the case of checking the relationship between the susceptibility of diabetes and SNP, the allele frequency for each SNP is statistically sorted between a group of diabetes patients and a control group. At this time, the control group used can be either a group of patients not having diabetes, or an average sample group extracted at random (regardless of whether or not the patients have diabetes or not).

In the case of specifying SNP related to the drug responsiveness of a newly developed drug, analysis beginning from step 1 is performed for a group for which the drug was effective or had side effects, and a group for which the drug was not effective or did not have side effects.

When there is an average sample group that was extracted at random, or when it is possible to use outside SNP data that corresponds to these groups, together with the aforementioned two groups, it is possible to compare and analyze data between a total of three groups and thus more effectively perform analysis.

### (Pg. 9)

(c) Step 1 (Determining the 'scanning domain' (base sequence domain): In this process model, by repeating the process from this step 1 to step 5 (to be explained later) as one cycle, the 'scanning domain' is gradually narrowed down from an initially large 'scanning domain' to a more localized 'scanning domain'. In the last cycle, by analyzing all of the SNP typing data existing in the 'scanning domain', the 'target' SNP is finally specified. First, the scanning domain is determined (step S3). This 'scanning domain' is a domain that is checked (scanned) for the existence of a 'target' SNP and is a continuous domain of a human genome base sequence. The physical length is variable as it is gradually narrowed down.

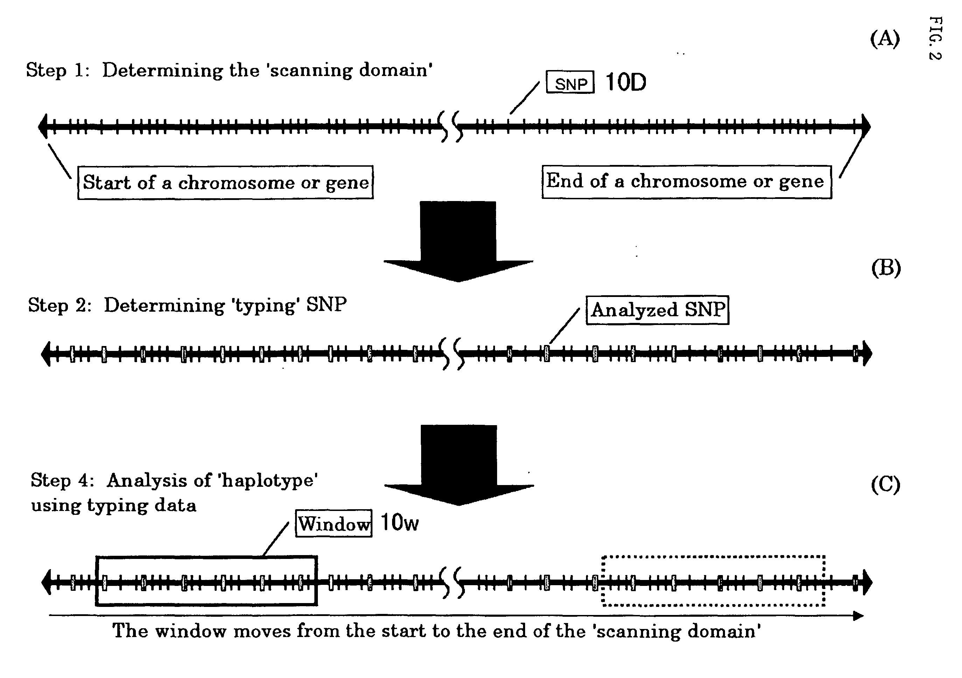

Figs. 2A to 2C are drawings to explain steps S3, S4 and S6 in Fig. 1. The steps in the processing cycle will be explained in more detail with reference to Fig. 1 and Figs. 2A to 2C.

Fig. 2A shows an example of a genome domain that contains SNP 10D in step S3 in Fig. 1. In step S3, the first 'scanning domain' is regulated (set) by a large level such as genes or even larger chromosomes. This is because even in this state large functions on the chromosome level are clear. Also, this includes the method of analyzing all of the chromosomes as the 'scanning domain' in the case when a plurality of chromosomes are the cause and it is not clearly known which chromosomes are suspect (include the target SNP), or in the case of taking all of the chromosomes except for certain chromosomes as the object (when there is no difference in the result between male and female, the sex chromosomes are meaningless so measures are taken such as to remove them from the 'scanning domain'), or as an extreme example, in the case when there is absolutely no information related to narrowing down the target SNP. Moreover, more specifically, it is possible to set the initial 'scanning domain' at the gene level for example. Or in other words, the scanning domain (primary scanning domain, initial scanning domain) is set based on a chromosome level for which the functions are known in advance.

### (Pg. 10)

In the second cycle ((n + 1)th cycle on, where n is a positive integer 1 or greater), the process returns again to step 1 (step S3) from step 5 (step S7) of the first (nth) cycle, so the domain in which chain imbalance was found in step 5 in the nth processing cycle (nth processing cycle) is set as the new 'scanning domain ((n + 1)th scanning domain)' in the (n + 1)th processing cycle. At this time, the narrowed down new 'scanning domain ((n + 1)th scanning domain)' has a length that is a large fraction down to a small fraction of the 'scanning domain (nth scanning domain)' of the previous (nth) cycle. How much the domain is narrowed down in the next cycle depends on the intensity of the chain imbalance between SNP (described later).

(d) Step 2 (Determining the typing SNP): Selects a group of SNPs for typing from among the 'scanning domain' set in step 1.

Fig. 2B shows an example of an SNP group that was selected from Fig. 2A. The SNPs contained in this group can be arbitrarily selected (without paying attention to separation according to gene site function or exon-intron), however, the SNPs should be selected such that the interval between SNP positions is as uniform as possible. This is in order to be able to indirectly observe chain imbalances between SNP positions in this series of analyses, and in order to be able to remove errors due to differences in physical distance between SNP positions that have a large effect on chain imbalances.

Chain imbalances between SNPs that can be analyzed are thought to occur when the physical distance between SNP positions is about 10,000 to 100,000 nucleotide bases. Therefore, when the SNPs contained in the first 'scanning domain' cover the entire length of the approximately 100,000 chromosomes, it is preferred that the first typing be performed for about 1,000 SNPs.

From the second processing cycle on, the 'scanning domain' is gradually narrowed down, and the physical distance of the 'scanning domain' becomes shorter, and thus the number of SNPs contained in this range decreases. The typing SNPs are selected in a range from several ten to several hundred from this 'scanning domain'.

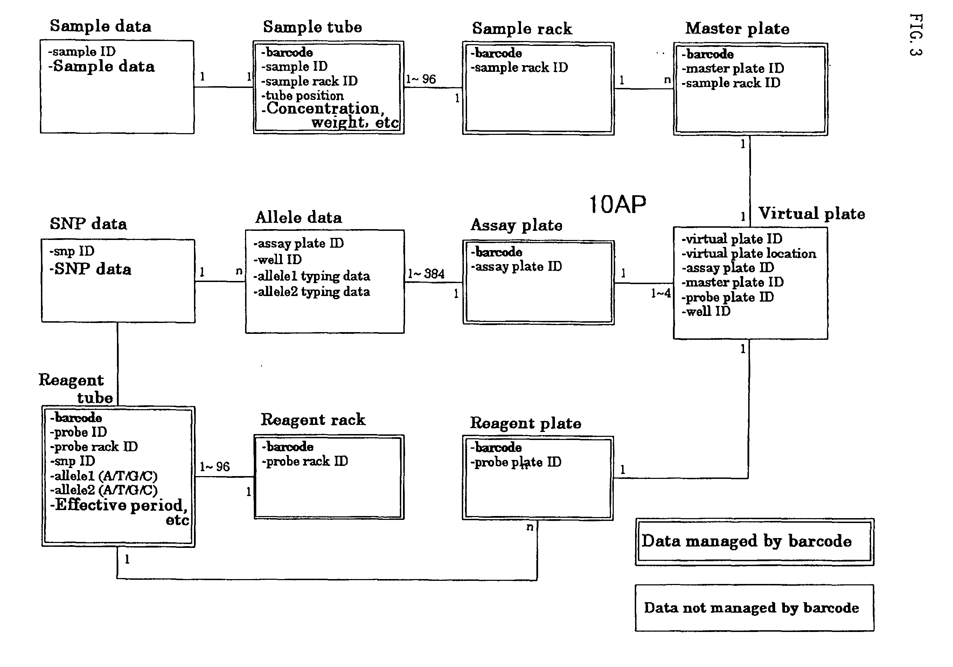

Fig. 3 is a drawing showing an example of the sample and reagent tubes, various plates and related data schemes for step S5 in Fig. 1.

### (Pg. 11)

(e) Step 3 (SNP typing by a wet process): Performs the SNP typing by a wet process shown in Fig. 1 (step S5). In other words, SNP typing of each sample is performed for the selected SNP group by the TaqMan PCR method or the like. In order to prevent data errors due to contamination or handling of the samples during this typing process, quality of the typing data is assured by performing, 1) generation management of the sample and reagent tubes and assay plates using barcodes, and 2) inspection of typing data using 'Hardy-Weinberg Equilibrium'.

1) Generation management of the sample and reagent tubes and assay plates using barcodes: The most common mistake made in the typing process is mishandling of samples and reagents. In the current SNP analysis process, some intermediate plates are created up to finally creating the assay plate 10AP that will be used in the typing apparatus for performing the assay. Therefore, it is important that these plates also be managed such that the plates are created from proper plates.

Therefore, together with using barcodes to perform ID management of the plates created by dispensing from the sample and reagent tubes, generation management is performed between tube and plate, or plate and plate to control the relationship of which was created as a parent and which was created as a child.

Sample: The sample is managed by sample tubes marked underneath with a 2-dimensional barcode, and by a sample rack that can store up to 96 of these sample tubes. The arrangement of the sample tubes in the sample rack is managed as data by reading the barcodes on the sample tubes from underneath the sample rack with a scanner. Also, a barcode is added to the sample rack itself.

Reagent: The reagent is managed by reagent tubes marked underneath with a 2-dimensional barcode, and by a reagent rack that can store up to 96 of these reagent tubes. Similar to the sample tubes, the arrangement of the reagent tubes in the reagent rack is managed as data by reading the barcodes with a scanner, and a barcode is added to the reagent rack itself.

### (Pg. 12)

Plates: There are three kinds of plates: master plates, reagent plates and assay plate 10AP. A master plate has 96 wells per plate (depressions where the sample or reagent is dispensed), and the sample is dispensed onto the plate from a sample rack that is similarly capable of storing a maximum of 96 sample tubes. At this time, one sample is dispensed to the well at one location (the wells on the master plate are positioned using the same layout at the sample tubes in the rack). Therefore, the master plate is managed as 'child' data of the sample rack. A reagent plate also has 96 wells (similar to a master plate), and since only one kind of reagent is use for each plate, the reagent plate is managed as 'child' data of the reagent tubes. The reason for keeping arrangement data for the arrangement of the reagent tubes in the reagent rack is because the automatic apparatus used for dispensing requires the use of reagent tubes stored in a rack. The assay plate 10AP has 384 wells, making it possible to perform simultaneous typing of 4 SNP for a maximum of 96 samples. This one assay plate 10AP is managed as four virtual plates in the data. One virtual plate is a plate used in performing typing of one SNP for a maximum of 96 samples, and one assay plate 10AP virtually comprises four of these plates.

2) Checking typing data using 'Hardy-Weinberg Equilibrium': A SNP has two variations of nucleotide bases for the SNP position, for example, it has two alleles (allelic genes) such as A (adenine) or G (guanine). All of the chromosomes are paired, so the SNP positions exist at a total of two locations for each of the paired chromosomes. Therefore, there are the following three SNP patterns that are observed in the TaqMan assay: 1) Homozygote A-A of a one-sided allele (in this case, A), 2) Homozygote G-G of a different allele (G) and 3) Hetrozygote A-G, which is a combination of opposing allele. By taking the probability that the SNP for one gene will have A to be α, then the probability of an A-A homozygote is α², the probability of a G-G homozygote is (1-α)², and the probability of an A-G hetrozygote is 2α (1-α), and this relationship is called 'Hardy-Weinberg Equilibrium'. The condition for establishing 'Hardy-Weinberg Equilibrium' is that data must be extracted at random from a sample group that is in equilibrium after several generations of hybridization, and at the same time must be statistically extracted by average.

### (Pg. 13)

Here, when the value of the obtained sample data greatly deviates from the 'Hardy-Weinberg Equilibrium', then it can be considered that either (1) that data is contaminated data due to contamination of the assay that generated the data, or (2) the typed sample group was not statistically extracted at random.

The object of SNP function analysis is to compare SNP data between sample groups that are separated according to whether or not they express a certain characteristic (disease, etc.), and to specify the SNP that is the cause for expressing that characteristic. In other words, when the SNPs that are the cause are typed, since there are more SNPs than expected that are causally related to the characteristic of that group, then it must have an allele distribution that deviates from the 'Hardy-Weinberg Equilibrium'. In other words, in the checking of typing data using 'Hardy-Weinberg Equilibrium', finding the corresponding SNP is the object of the SNP function analysis and this process model.

In order to specify the 'target' SNP, the SNPs having 'contaminated' data in (1) are removed from the SNP of the allele distribution that deviated from the 'Hardy-Weinberg Equilibrium'.

The method of identifying the 'contaminated' data due to this contamination is a method of typing four SNPs using one assay plate 10AP.

### (Pg. 14)

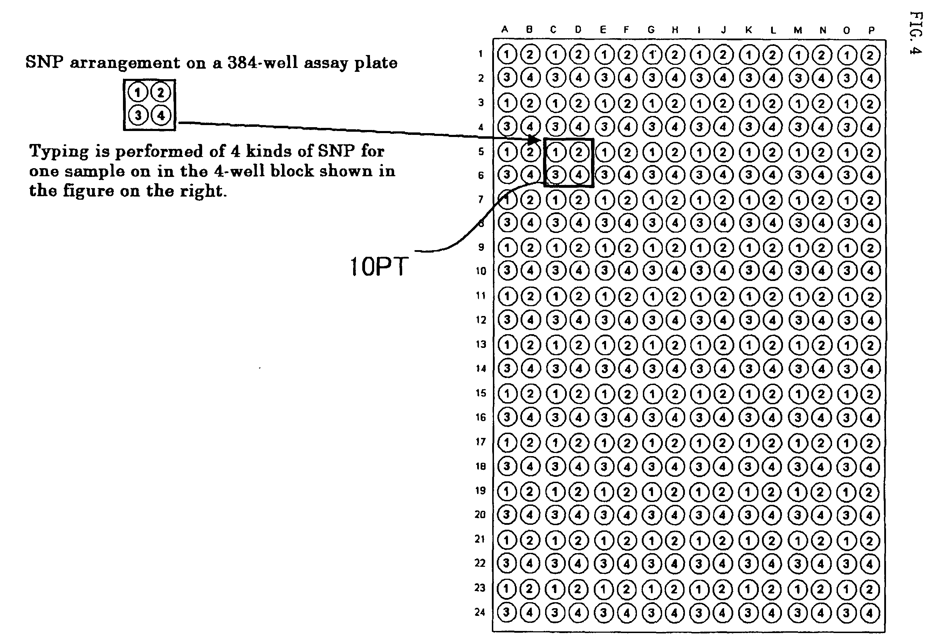

Fig. 4 is a drawing showing an example of the arrangement pattern of SNPs on the assay plate 10AP of Fig. 3. The assay plate 10AP has 384 wells, and the layout of the typing SNPs is as shown in Fig. 4.

When there is failure of the assay itself due to contamination or problems in the PCR method, it is considered that problems will also occur for other SNPs on the same plate.

Since the chance of being able to select a plurality of SNPs that are the cause is thought to be extremely rare, in the case that a plurality of SNP data for the same plate deviates from the 'Hardy-Weinberg Equilibrium', typing is determined to have failed, and all of the data for that plate is discarded (or the assay is redone).

Furthermore, in order to avoid the possibility of chain imbalances between typed SNP pairs, the four SNPs are separated as much as possible, or in other words, the 'scanning domain' is divided into four sections 10PT (four well blocks), and one SNP to be typed is selected from each respective section.

Fig. 2C shows an example of the scanning domain for step S6 in Fig. 1. In the processed cycle, the window 10w is moved from the start of the ' scanning domain' to the end, and an image of analyzing the SNP data contained in that window 10w is shown.

(f) Step 4 (haplotype analysis using typing data, step S6): In this process, the two concepts, haplotype and chain imbalance, are used to specify an SNP (that will in the end become the 'target' SNP itself) near the 'target' SNP.

The haplotype, which is a combination of opposing genes (SNP allele) on one gamete (one of the paired chromosomes), is stochastically predicted based on data obtained from the SNP typing assay using the TaqMan method.

This stochastic prediction of the haplotype will be considered from the case of haplotypes that are taken by the following three SNP; SNP#1 that takes A or G, SNP#2 that takes T or G and SNP#3 that takes T or C.

### (Pg. 15)

For example, for a sample X, in the case where SNP#1 takes the homozygote A-A, SNP#2 takes the hetrozygote T-G and SNP#3 takes the hetrozygote T-C, it is predicted that four haplotypes exist for the following two cases.

| Case 1) | SNP#1 | SNP#2 | SNP#3 | Probability |
|---|---|---|---|---|
| Chromosome 1 | A | T | T | 25% |
| Chromosome 2 | A | G | C | 25% |
| | | | | |

| Case 2) | SNP#1 | SNP#2 | SNP#3 | Probability |
|---|---|---|---|---|
| Chromosome 1 | A | T | C | 25% |
| Chromosome 2 | A | G | T | 25% |

In this example, the probability for each haplotype is 25%.

Furthermore, in another sample Y, in the case where SNP#1 takes the homozygote A-A, SNP#2 takes the hetrozygote T-G and SNP#3 takes the homozygote T-T, it is predicted that two haplotypes exist with each having a probability of 50%.

| Case 1) | SNP#1 | SNP#2 | SNP#3 | Probability |
|---|---|---|---|---|
| Chromosome 1 | A | T | T | 50% |
| Chromosome 2 | A | G | T | 50% |

Also, the haplotypes predicted from these two samples are as shown below.

| SNP#1 | SNP#2 | SNP#3 | Probability |
|---|---|---|---|
| A | T | T | 25%/2 + 50%/2 = 37.5% |
| A | T | C | 25%/2 = 12.5% |
| A | G | T | 25%/2 + 50%/2 = 37.5% |
| A | G | C | 25%/2 = 12.5% |

In the actual analysis, a continuous domain that contains a certain number of SNPs specified by a range of several to several tens of SNPs is defined as the window 10w, and the combinations and the probability of each haplotype emerging from the typing data of the SNPs in that window 10w (for all samples) is found statistically. When the number of SNPs contained in this window 10w is too large, the probability for each haplotype decreases and it becomes difficult to confirm the existence of chains or chain imbalances, so it is effective to define the window 10w such that it contains about 10 SNPs.

### (Pg. 16)

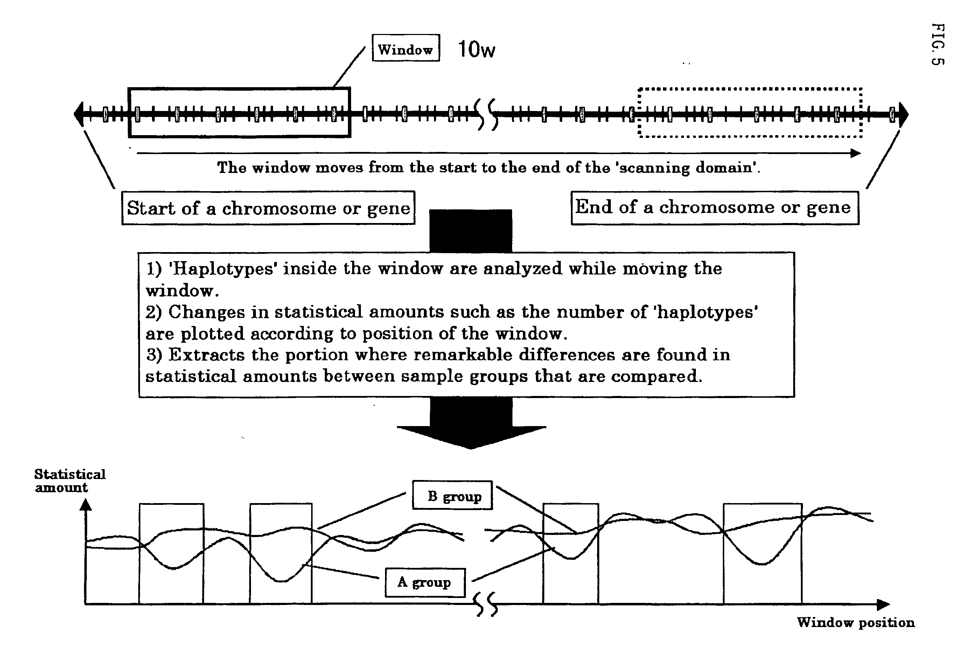

Fig. 5 is a drawing that shows an example of the analysis data for step S7 (step 5) in Fig. 1.

Fig. 5 shows the procedure for identifying domains in the analysis data in which changes occur in the statistical amount from haplotype analysis for the first half of the inspection.

(g) Step 5 (estimating the 'marker' SNPs, step S7): In this process, as shown in Fig. 5, domains near the 'target' SNP are estimated by finding domains in which the probability of a certain haplotype stands out. In other words: (1) The haplotypes in the window 10w are analyzed while moving the window 10w. (2) Changes in statistical amounts such as the number of ' haplotypes' according to the position of the window 10w are plotted. (3) Areas where remarkable differences are found in the statistical amounts between sample groups that are compared are extracted.

It becomes possible to determine whether or not chain imbalances are seen between analyzed SNP groups from the haplotype data that were analyzed in step 4.

When no chain imbalances are seen between these SNPs, it is thought that the frequency of appearance of each SNP allele will become steady at an 'average' value, and since each of the SNPs is 'independent' , the haplotypes that are statistically found from will not be concentrated at a certain haplotype, but will be widely and thinly dispersed.

On the other hand, when chain imbalances are seen between the analyzed SNP groups, then SNPs that statistically characterize the sample groups are contained in the groups, and the frequency of appearance of a certain allele in those SNPs increases. Also, it is predicted that the probability distribution of the haplotypes that are the results of the statistical analysis of these SNP data will concentrate on a certain haplotype (when assay is not performed for the 'target' SNP).

### (Pg. 17)

As the method of identifying the concentration at this certain haplotype, besides comparing the frequency of appearance of each individual haplotype, the total number of haplotypes predicted from that analysis data, the standard deviation of these haplotypes, and the ratio of the frequency of appearance with respect to all of the haplotypes of the upper probability haplotype group are observed as the 'statistical amount', and these are compared between sample groups that are separated according to the expression of the characteristic of whether or not a drug is effective or has side effects.

However, as described above, it is very difficult to select and directly type a 'target' SNP. This problem is solved by applying the chain imbalance between the 'target' SNP and the nearby SNPs, and by estimating the domain near the 'target' SNP. The nearby SNP with the chain imbalance is weak when compared with when the 'target' SNP is analyzed directly, and similarly, it is expected that the probability distribution of the haplotype will change. This kind of nearby SNP is considered to be a ' marker' SNP for the 'target' SNP. In other words, the statistical amount of the sample that is the object of analysis (group with effect: Case group) is compared with the reference statistical amount of the reference sample (group having no effect: Control group) and when the difference exceeds a preset threshold value, it is determined that there was change in the corresponding typing domain (estimated as the marker SNP), and a specified ratio (for example 1/5 to 1/10) with respect to that typing domain is set as a new scanning domain, and the next processing cycle is performed.

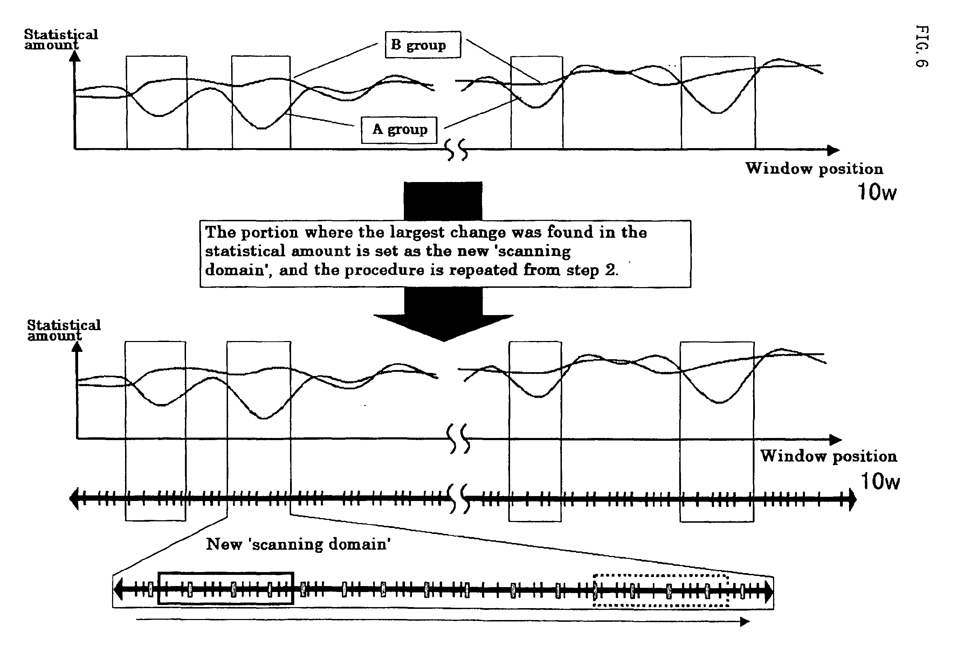

Fig. 6 is a drawing that shows an example of the analysis data in step S7 shown in Fig. 1. Fig. 6 shows the procedure for determining the new 'scanning domain' for the later half of the inspection of analysis data in step 5.

A domain where a certain haplotype stands out is found from the typing data of the 'marker' SNP without directly typing the 'target' SNP, and the process returns to step 1 to set that domain as the next 'scanning domain' , and the process is repeated.

Also, in that cycle, after all of the SNPs in the 'scanning domain' are typed, the process advances to the next step 6, and the SNP positions that converge at that certain haplotype are set as the 'target' SNP.

### (Pg. 18)

The purpose of this step is to estimate the 'marker' SNPs, and by repeating the cycle of narrowing down the 'scanning domain' near the newly estimated 'marker' SNP, it can be said that it is possible to bring the 'marker' SNP close to the 'target' SNP. Even though the 'target' SNP may be derived from analysis before the last cycle, at that point it is still not possible to set that SNP as the 'target' SNP, so it becomes the 'marker' SNP.

(h) Step 6 (specifying the 'target' SNP, step S8): The purpose of this process is to correlate the 'target' SNP that is selected from the overall process with the drug responsiveness of the newly developed drug, and quantitatively derive the degree of correlation.

In this process, a final inspection is performed in the sample group for the allele frequency of the SNP that is set as the 'target' SNP. (A Chi-square test or method of maximum likelihood is used.)

Furthermore, it is effective to perform a comparison with SNP data other than that of the sample group, particularly data belonging to the ' The Institute of Medical Science, The University of Tokyo' that is related to SNP representative of the Japanese people. Moreover, there is a movement in some genome research businesses to sell databases related to SNP, so these can also be used.

Evaluation tests are performed using a Chi-square test or correlation analysis to evaluate whether the expected results are obtained at a certain percent of probability, or whether severe side effects occur at a certain percent of probability.

Here, the method of identifying contaminated data will be explained in detail.

1) SNP allele distribution in a plate that was set according to the typing results (Typing is performed for four kinds of SNP out of a maximum 96 (actually up to 92) on one plate, however, this means the allele distribution for each SNP.)

Here, the 'allele distribution' is the total number of samples that take one of the three values in the group of SNP typing data (SNP typing data of the sample for which typing was performed for the same SNP on the same plate) expressed by any of three values, such as AA/AB/BB or AA/Aa/aa. (For example, AA:23, AB:54, BB:15)

### (Pg. 19)

2) Ideal SNP allele distribution based on Hardy-Weinberg Equilibrium: 'Hardy-Weinberg Equilibrium' is said to be the state where when the frequency of 'AA' is taken to be α², the frequency of 'BB' is (1 - α)², and the frequency of 'AB' is 2α(1 - α)².

(Here, [frequency of 'AA'] > [frequency of 'BB'])

(The probability of a single 'A' (= frequency) is α, so when both are 'A', the frequency 'AA' becomes α², the probability of 'B' that is not the probability of 'A' is 1 -α, the probability of 'BB' is (1 - α)², and the probability of 'AB', which is the total probability of the two cases 'A' x 'B' and 'B' x 'A', is 2α (1 - α)².

The 'ideal SNP allele distribution based on Hardy-Weinberg Equilibrium' is calculated with the frequency of 'AA' taken to be α², and the frequency of the remaining 'BB' and the frequency of 'AB' being calculated as (1 - α)² and 2α (1 - α)², respectively. In other words, in example 1), the frequency of 'AA' is 23/96 = 25%, and the probability of a single 'A' becomes 50%. Therefore, the frequency of 'BB' is also 25%, and the allele distribution is 96 x 25% = 23, the frequency of 'AB' is 50% and the distribution is 96 x 50% = 46, and becomes the 'ideal SNP allele distribution based on Hardy-Weinberg Equilibrium'.

3) Comparison of the aforementioned two allele distributions: The two SNP alleles of 1) and 2) are checked by a Chi-square test whether or not they have a 'significant statistical difference'.

In the previous examples, AA, AB and BB are as follows.
1) 23, 54, 15
2) 23, 46, 23

Here, the result of the Chi-square test is 12.41%, which is greater than the value (probability less than 5%) recognized as a 'significant statistical difference', so it is determined that there is no significant difference.

### (Pg. 20)

After identifying contaminated data in this way:

4) When significant difference is found in the comparison results, and when it is found that there is a 'significant statistical difference' in the result of 3) for two or more SNPs that were simultaneously typed on that plate, typing of that entire plate is determined to have failed, and that typing data is discarded.

5) When 'significant statistical difference' is found in only one SNP of the SNPs on the same plate, typing for just the SNP for which the significant difference was found is performed again. (When the same analysis result is obtained, that typing data is stored as being 'correct'. When the 'significant difference' in the re-executed typing result is not removed, typing is performed for a third time. The procedure below is repeated, and typing is performed until the same result is continuously obtained.)

The method of specifying SNP of a first embodiment of the invention is as was described above, and it has the following effect.

When identifying an SNP from among the typed SNP that is related to disease susceptibility or drug responsiveness, by gradually narrowing down the base sequence domain that is the object of the analysis from a large domain to a more localized domain, and by performing typing of the SNP using a quality controlled process, it is possible to finally identify a related SNP.

### (Embodiment 2)

Next, a method for specifying SNP of a second embodiment of the invention will be explained.

When determining the 'scanning domain' in step 1 (step S3 in Fig. 1), it is possible to set the initial 'scanning domain' on the genetic level, which is more detailed than the larger chromosome level. In this way, typing can be started from a narrow initial 'scanning domain', so the 'scanning domain' is more effective.

When setting the 'typing' SNP in step 2 (step S4 in Fig. 1), when the interval between observed SNP positions is too large (particularly in the early stages of the 'scanning domain'), it is almost impossible to observe any chain imbalance. It is necessary to select 'typing' SNP have an interval that is shorter than the physical distance of the level in which it is possible to observe this chain imbalance.

### (Pg. 21)

In the initial 'scanning range', this interval is such that one SNP is selected for 1,000 SNP. In other words, the initial value is set such that one SNP is selected for 100,000 nucleotide bases. However, this just a calculated value, and depending on the degree of the chain imbalance seen near that, there are case in which a smaller value is taken (one SNP is selected for several tens of SNPs).

In the estimation of the 'marker' SNP in step 5 (step S7 in Fig. 1), beside the method of comparing changes in 'statistical amounts' such as the total number or frequency of appearance of haplotypes, it is possible to determine whether or not there is a chain or chain imbalance by finding the SNP pattern that is common with the haplotypes having a high level of probability.

In the example of haplotype probability explained in the first embodiment, the sum of the haplotype probabilities for sample X and sample Y were as shown below.

| SNP#1 | SNP#2 | SNP#3 | Probability |
|---|---|---|---|
| A | T | T | 25%/2 + 50%/2 = 37.5% |
| A | G | T | 25%/2 + 50%/2 = 37.5% |
| A | T | C | 25%/2 = 12.5% |
| A | G | C | 25%/2 = 12.5% |

In this example, for this group of haplotypes it is shown that SNP#1 takes allele A and SNP#3 takes allele T at a probability of 75%, so it can be estimated that there is link between SNP#1 and SNP#3.

Also, in a more complicated example:

| SNP#1 | SNP#2 | SNP#3 | SNP#4 | SNP#5 | Probability |
|---|---|---|---|---|---|
| A | T | T | A | T | 30% |
| A | T | T | A | G | 25% |

| | | | | | |
|---|---|---|---|---|---|
| C | T | T | A | T | 10% |
| C | T | T | A | G | 10% |
| A | T | T | C | T | 5% |
| A | T | T | C | G | 5% |
| A | G | C | A | T | 3% |

### (Pg. 22)

A haplotype pattern is observed in which SNP#1 has allele A, SNP#2 has allele T, SNP#3 has allele T and SNP#4 has allele A at a probability of 55%. Furthermore, a haplotype pattern is observed in which SNP#2 has allele T, SNP#3 has allele T and SNP#4 has allele A at a probability of 75%, and a haplotype pattern is observed in which SNP#2 has allele T and SNP#3 has allele T at a probability of 85%. From this it is determined from the data of this sample group that a chain can be seen centered on SNP#2 and SNP#3.

The threshold value of the appearance probability that divides whether or not there is a chain is related to the number of SNP scanned in the window 10w. As the number of SNP increases, the variations of haplotypes increases, and the probability that each individual haplotype will be observed is decreased, so as a result, the threshold value also becomes lower. When a window 10w in which 10 SNP are observed is used, a threshold value of 70% is considered to be appropriate. The domain (window size) scanned by the window 10w varies according to the object, however, about 3 to 25 SNP is generally considered to be appropriate.

The method of specifying SNP of a second embodiment of the invention is as described above, and has the following effect in addition to the effect of the first embodiment.

It is possible to specify the 'target' SNP or the domain near it by determining whether there are different chains existing between two sample groups that are compared, or whether there is a chain that exists in only one sample group.

The embodiments were explained using haplotype analysis, however, it is evident that general statistical analysis could also be used. Also, the invention is not limited to that, and when applying this invention it can be applied to a suitable SNP specifying method. Moreover, in order to realize the embodiments, it is possible to construct a system for specifying SNP that comprises a chromosome-level or DNA-level SNP typing process apparatus and a computer that performs statistical analysis, and that is capable of performing a series of processes. Also, the number, position, shape of the components are not limited to those of the embodiments described above, and the invention can be embodied by using any suitable number, position or shape. In the drawings, the same reference numbers are used for identical component elements.

### Industrial Applicability

The present invention is constructed as described above so has the following effect.

As was explained above, with this invention, by estimating a SNP to be a marker, the base sequence domain that is the object of analysis is gradually narrowed down from a large domain to a more localized domain (statistical amounts for patients and non patients are compared and the SNP domain is narrowed down), and furthermore, by typing the SNP using a quality controlled process, it is possible to finally specify SNP related to disease susceptibility or drug responsiveness.

## Claims

1. A method of specifying SNP related to disease susceptibility or drug responsiveness and comprising:
a first step of setting a scanning domain beforehand in the base sequence domain that is the object of SNP analysis;
a second step of gradually narrowing down said scanning domain to a localized domain that contains a target SNP; and
a third step of specifying said target SNP from said narrowed down localized domain.

2. The method of specifying SNP of claim 1 wherein said second step comprises a step of setting a marker SNP for specifying said target SNP and gradually narrowing down said scanning domain.

3. The method of specifying SNP of the second claim wherein said second step uses statistical analysis such as haplotype analysis to set said marker SNP.

4. The method of specifying SNP of claim 3 wherein
said first step comprises: a step of setting the scanning domain of said base sequence domain in a genome domain that is limited to genes whose functions are clearly known or chromosomes whose functions can be predicted; and
said second step comprises:
a fourth step of selecting a group of SNP to be typed from said scanning domain and performing SNP typing using a wet process;
a fifth step of finding the probability of appearance of all combinations of said haplotype analysis in said scanning domain based on typing data of said SNP typing as a statistical amount; and
a sixth step of comparing the found said statistical amount with a preset or estimated reference statistical amount, and when there is significant deviation between said statistical amount and said reference statistical amount that exceeds a preset threshold, determining that said marker SNP is contained in the domain corresponding to the deviated position that exceeds said threshold value.

5. The method of specifying SNP of claim 4 wherein said third step comprises:
a seventh step of increasing the specified ratio of the number of SNPs to be the object of typing in the selection of the SNP group in said fourth step when said significant deviation is less than a first threshold value, and then repeating said fifth step;
an eighth step of setting a new scanning domain from said scanning domain that has been decreased by a specified ratio such that it contains the position of the deviated peak when said significant deviation is greater than said first threshold value but less than a second threshold value, and then repeating said fifth step; and
a ninth step of determining that said marker SNP is contained in the domain corresponding to the deviated position that exceeds said second threshold value when said significant deviation exceeds said second threshold value, setting a new scanning domain from said scanning domain that has been decreased by a specified ratio such that it contains the position of the deviated peak, and then repeating said fifth step.

6. The method of specifying SNP of claim 5 wherein said ninth step comprises a step of setting SNPs that include the target SNP for which all DNA samples are typed when the number of SNPs in a selected group is less then a specified number.

7. The method of specifying SNP of claim 5 wherein said seventh step comprises a step of determining that the target SNP is not contained and stopping the process when the number of times the process of said fifth step is performed exceeds a specified number of times.

8. The method of specifying SNP of claim 5 in which said eighth step comprises a step of determining that the target SNP is not contained and stopping the process when the number of times the process of said fifth step is performed exceeds a specified number of times.

9. The method of specifying SNP of any one of the claims 1 thru 8 wherein said second step comprises a step of typing the SNP using a quality controlled process, and where said quality controlled process performs typing of four SNP on one assay plate for one sample, and determines that the typing data is invalid when the number of typed SNPs found having significant difference by a statistical method such as the Chi-square test exceeds a specified number and identifies the data as being contaminated by contamination of the sample.

10. The method of specifying SNP of claim 9 in which said second step repeats SNP typing only for SNP found to have said significant difference when the number of typed SNPs found to have significant difference was a specified number, and when the result of no significant difference continues for a specified number of times, determines that said typing data is correct and uses that data.

11. A computer program that can be read by a computer that can execute the processing of the method of specifying SNP of any one of the claims 1 thru 10 wherein all of the steps of any one of the claims 1 thru 10 are coded.
